# EUROPEAN PATENT APPLICATION

(11) **EP 1 158 057 A1**
(43) Date of publication of application: **28.11.2001**
(21) Application number: 00401356.1
(22) Date of filing: 18.05.2000
(51) Int. Cl.: C12Q 1/68

(54) **Compositions and methods applicable to genetic analyses**

(71) Applicant: CENTRE NATIONAL DE LA RECHERCHE SCIENTIFIQUE, 75016 Paris Cédex (FR)
(72) Inventor: Dumas, Sylvie, 75006 Paris (FR); Mallet, Jacques, 75013 Paris (FR)
(74) Representative: Becker, Philippe

(57) **Abstract**

The present invention relates to compositions and methods for genetic analyses. More particularly, this invention provides compositions and methods for differential gene expression analyses on biological material, such as tissue sections. This invention discloses more preferably differential gene expression analyses on biological material using particular probes with distinct radioactive labels. The present invention can be used to detect or monitor gene expression, compare gene expression (e.g., differential gene expression screening) in particular in different tissues, and is suitable for instance in research, diagnostic, and many pharmacogenomics applications.

## Description

### FIELD OF INVENTION

The present invention relates to compositions and methods for genetic analyses. More particularly, this invention provides compositions and methods for differential gene expression analyses. Even more particularly, the invention provides compositions and methods for analysing gene expression on biological material, such as tissue sections. This invention is based on hybridisation between the biological material and particular probes, more specifically in situ hybridisation with radioactive probes. This invention discloses more preferably differential gene expression analyses on biological material using particular probes with distinct radioactive labels. The present invention can be used to detect or monitor gene expression or to compare gene expression (e.g., differential gene expression screening), for instance, and is suitable for use in research, diagnostic and many pharmacogenomics applications, for instance.

### BACKGROUND

Various methods of genetic analysis or target nucleic acid detection have been described in the art, based on hybridisation with probes specific for the target gene (or nucleic acid). Such methods essentially comprise contacting a biological sample to be analysed with the probe, under conditions allowing nucleic acid hybridisation, and detecting the formation of hybrids, as an indication of the presence of the target nucleic acid in the sample. These techniques have been used to detect the presence of a nucleic acid, to monitor gene expression and/or regulation, to compare gene expression in different samples, etc.

In this regard, The *in situ* hybridisation method (ISH) is a common procedure for the detection of genetic material. It reaches a large number of biological fields such as anatomy, cellular biology and gene expression regulations. Since 1990, the characterisation of numerous genes and cDNAs but also the rapid development of molecular biology techniques has allowed the diffusion, refinement and user friendliness of ISH. It has become of great importance as a powerful method for localizing individual cells that contain particular species of mRNA within complex, heterogeneous tissues, such as the nervous system for instance. The basics of in situ hybridisation have been described for instance in "*In situ hybridisation: a practical approach"* (D.G. Wilkinson ed., Oxford University Press, 1992) or in Leitch et al. ("*In situ hybridisation: a practical guide",* Macroscopy handbooks 27, 1994). The anatomical data provided by ISH are very accurate and allows the performance regional, cellular and sub-cellular patterns of gene expressions. However, these prior art techniques of gene detection or analysis suffer from several drawbacks. In particular, to this date, fluorescent labelling has been used to allow multiparametric detection of genes 'i.e., the simultaneous visualization of several genes). However, fluorescence does not allow quantification and is not sensitive enough to detect fine gene regulations or rare gene products. Furthermore, while quantitative data about the level of gene expression might be possible with the use of radioactive labelling, radioactive labelling has long been considered has unsuitable for frequent in situ hybridisation because of the technical difficulties inherent to radioactivity (security, length of acquisition, etc). Furthermore, such quantitative analyses were only achievable for one gene per experiment.

Therefore, it would be of major interest to gain the ability to routinely and precisely detect and quantify several mRNAs on the same tissue section and at a cellular level. It would be very advantageous to provide methods that would be sensitive, reliable, and allow detection and quantification of genes or gene products that are present or altered at low levels.

### SUMMARY OF INVENTION

The invention now provides methods and compositions for simultaneous detection and/or discrimination of target nucleic acids in a sample, using radioactive probes.

The invention provides methods and compositions for simultaneous visualization and/or quantification of several nucleic acids in a biological sample, using radioactive probes.

The invention more specifically uses several sets of radio-labelled probes that specifically hybridise with target nucleic acids and exhibit different (distinguishable) radiolabels.

The present invention discloses, for the first time, methods that allow co-detection and quantitative analysis of gene expression using radioactive probes. This invention more particularly discloses that it is possible to differentiate gene expression using radioactive probes on the same tissue sample, more particularly in the same cell.

The instant invention describes more specifically the simultaneous hybridisation and visualization of two radioactive probes on the same tissue section, each probe being labelled with different radio-elements (³³P/³⁵S/³H/³²P/¹²⁵I, etc.). Taking in consideration the specific activity difference between various radiolabelled nucleotides, the invention also discloses preferred methods and conditions allowing the use of these (five) different radioactive nucleotides to differently label different oligonucleotide probes that would be hybridised on the same tissue section and efficiently discriminate the probes on the same sample.

A particular aspect of this invention resides in a method of detecting target nucleic acids in a biological sample, comprising:
a) contacting the biological sample with at least two sets of radioactive probes, the probes of the first set being specific for a first target nucleic acid and labelled with a first radio-label, and the probes of the second set being specific for a second target nucleic acid and labelled with a second radio-label, and
b) detecting said first and second target nucleic acids in the biological sample by assessing the formation of hybrids between the probes and the sample.

In particular variants of this invention, the probes may be DNA molecules between 15 and 500 base-pairs-long, even more preferably single stranded DNA oligonucleotides between 15 and 500 bases long, or RNA molecules, between 15 and 3000 bases long.

In further particular variants, the probes may be labelled by (i) a 3' radioactive tracer, preferably a 3', 5-100 long, radiolabelled nucleic acid tail, (ii) a 5' radioactive tracer, and/or (iii) insertion in their sequence of radioactive nucleotides, i.e., comprise, in their sequence, radioactive nucleotides.

Particular ways of carrying out the instant invention comprise:
- using two sets of probes comprised of oligonucleotide probes,
- using two sets of probes comprising a 3' radioactive tracer,
- using a first and second radioelements having a different emission-energy spectra, preferably a first set of probes labelled with tritium and a second set of probes labelled with a radioisotope selected from ³⁵S, ³³P, ³²P and ¹²⁵I.

According to specific embodiments of the present invention, the two sets of probes are contacted simultaneously with the biological sample, preferably using sets of probes having a similar specific activity, and using essentially similar amounts of each set of probes.

According to other preferred aspects of the present invention, the biological sample is a mammalian tissue sample, preferably a tissue section, and several biological samples are tested in parallel, preferably after being deposited on one or several supports, preferably glass support.

In this regard, another object of this invention resides in a method of detecting target nucleic acids in several biological samples, comprising:
a) providing biological samples on one or several supports, preferably glass supports,
b) contacting, in parallel, the biological samples on the support(s) with at least two sets of radioactive probes, the probes of the first set being specific for a first target nucleic acid and labelled with a first radio-element, and the probes of the second set being specific for a second target nucleic acid and labelled with a second radio-element, and
c) detecting said first and second target nucleic acids in the biological samples by assessing the formation of hybrids between the probes and the samples.

An other specific object of this invention is a method of detecting target nucleic acids in a biological sample, wherein each biological sample is contacted, in parallel with the following at least two sets of probes:
a) probes of a first set specific for a first target nucleic acid and labelled with a first radio-label and probes of a second set specific for a second target nucleic acid and labelled with a second radio-label,
b) probes of the first set specific for the first target nucleic acid and labelled with the second radio-label and probes of the second set specific for the second target nucleic acid and labelled with the first radio-label,
and wherein the method further comprises assessing the formation of hybrids between the probes and the samples.

Still a further object of this invention lies in a method for comparing target gene expression in at least two biological samples, comprising:
a) contacting, in parallel, the biological samples (preferably on one or several supports) with at least two sets of radioactive probes, the probes of the first set being specific for a first target nucleic acid and labelled with a first radio-element, and the probes of the second set being specific for a second target nucleic acid and labelled with a second radio-element,
b) assessing the formation of hybrids between the probes and the samples, and
c) comparing target gene expression in said samples by comparing the relative amount of hybrids formed between the samples.

Further aspects of this invention include nucleic acid probes comprising radioactive nucleotides labelled with tritium, as well as an isolated nucleic acid molecule, wherein the molecule is single strand, comprises a 15-500 bases-long sequence, preferably 15-250, more preferably 15-100, which is complementary to a target nucleic acid, and comprises a 3' tritiated nucleotide tail.

This invention also relates to the use of two radioactive probes with different specificity and different radioactive label, for in vitro or ex vivo gene expression analysis on a biological sample.

The invention may further be used in combination with other labelled probes or detection reagents or techniques, in order to provide further detailed information about a tissue sample. Such additional probes or reagents include fluorescent probes as well as labelled antibodies, specific for proteins or polypeptides. In this respect, a preferred embodiment of the invention comprises the simultaneous detection target nucleic acids (using radiolabelled probe(s)) and target polypeptides (preferably using labelled antibodies, such as fluorescent-, enzymatic-, chemical- or radio-labelled antibodies).

In this regard, a particular variant of this invention resides in a method as defined above, further comprising contacting the biological sample(s) with a non-radioactive probe and/or an affinity reagent to detect additional target nucleic acid(s), polypeptide(s) or cellular component(s).

In a particular embodiment, a further detection reagent is a radiolabelled antibody, and the antibody is used in combination with a radiolabelled nucleic acid probe. More generally, this invention can be used for simultaneous detection (or quantification) of at least two target components of a cell or tissue (including nucleic acid, polypeptide, organelle) using two differently radiolabelled detection reagent (e.g., two nucleic acid probes, two antibodies, one nucleic acid probe and one antibody, etc.).

The invention also encompasses kits for nucleic acid (or other component) detection comprising radioactive nucleotides (or other detection reagents such as antibodies), enzymes and/or protocols for radioactive labelling of nucleic acid probes or antibodies as well as, more generally, any kit for implementing a method as defined above, comprising the reagents, supports and/or protocols for labelling, hybridisation and/or readout.

This invention can be used in many different technical areas, with virtually every type of biological material.

### DETAILED DESCRIPTION OF THE INVENTION

As indicated, the present invention resides in methods of detecting gene expression or regulation using particular probes. The present invention will now be disclosed in further details, the details being merely illustrative and not limiting the scope of the invention.

### The probe

The probe may be any nucleic acid molecule comprising a region of pre-determined sequence, more preferably any single-strand nucleic acid molecule comprising a region of pre-determined sequence. The region of pre-determined sequence comprises at least 15 consecutive nucleotides, more preferably at least 20 consecutive nucleotides. The sequence of this region is determined according to the target nucleic acid molecule which is to be detected or monitored in the biological sample. The target nucleic acid may be any (portion of a) gene, RNA, chromosome, viral genome, mitochondria, plasmid, episome, PNA (Peptide Nucleic Acid), etc. For instance, where a particular gene or RNA is to be detected or monitored, the sequence of the region is complementary to said gene or RNA, preferably perfectly complementary, in order to allow specific hybridisation therewith. Although perfect matching (or complementarity) is preferred, it should be understood that mismatches may be tolerated, as long as the probe can specifically hybridise with the target nucleic acid under appropriate stringency conditions. The probes can be designed to avoid likely regions homology amongst members of a family of gene transcripts or, conversely, they can be targeted against a conserved, usually translated region in order to detect the same gene transcript in various species, for instance. The probe may also be designed to hybridise with all splicing variants of a gene or, on the contrary, with only one particular splicing form of a selected gene. Furthermore, one of the sets of probes may be specific for a control reference nucleic acid.

The probe may a DNA molecule or an RNA molecule or a PNA molecule. In a particular embodiment, the probe is single- or double-strand DNA molecule between about 15 and about 2000 base(-pair) long, more preferably between 15-500. In a preferred embodiment, the probe is a single-strand DNA molecule, such as an oligonucleotide, comprising between 15 and 500 nucleotides, preferably between 15 and 100 nucleotides, more preferably between 20 and 50 nucleotides, even more preferably between 25 and 50 nucleotides. It should be understood that the size of the oligonucleotide probe may be adapted by the skilled person, and may be possibly larger than above indicated. Preferably, the size should allow specific hybridisation of the probe with a target nucleic acid, and thus include at least 10 or 15 nucleotides. The oligonucleotide may be produced according to conventional techniques, such as through DNA synthesizer, by any synthetic or semi-synthetic method, DNA cloning, digestion, ligation, and the like. Furthermore, the oligonucleotide may contain modified bases or may be further modified in order to increase its stability, or the stability of the hybrid, for instance. Such modifications include chemical modifications, enzymatic modifications, etc. In particular, the oligonucleotide probe may comprise modified nucleotides (e.g., biotinylated), modified bounds (phosphorothioates, etc.), intercalating agents (ethidium, etc.), etc.

The probe can also be a single-strand RNA molecule, comprising between 15 and 3000 nucleotides, more preferably between 20 and 2000 nucleotides, even more preferably between 25 and 1000 nucleotides. The RNA molecule may be produced according to conventional techniques, such as through RNA synthesizer or, preferably, by in vitro transcription from a DNA sequence encoding the same. This production method is preferred since it allows the production of large amounts of long (i.e., above 3000 nucleotides long) RNA probes. If needed, the RNA molecule may be further modified in order to increase its stability, or the stability of the hybrid, for instance. Such modifications include chemical modifications, enzymatic modifications, etc.

### Labelling

As indicated above, this invention resides in the use of radioactive probes, more specifically probes having distinct radioactive labels, in order to detect and monitor fine gene expression and regulation within biological samples. More specifically, the invention resides in the use of at least two sets of probes having a different radioactive label, the probes of the first set being specific for a first target nucleic acid and the probes of the second set being specific for a second target nucleic acid.

### . Radiolabel

As indicated, the invention uses at least two sets of probes which are differently radiolabelled. Preferably, each set of probes contains probes labelled with one particular radioelement, which can be distinguished from the radioelement used for the other set(s) of probes.

In this regard, many radio-elements or isotopes can be used for the labelling of the probes. Specific examples of isotopes include ³H, ³⁵S, ³³P, ³²P, ¹⁴C, ¹²⁵I, and the like.

Preferably, the invention uses at least two sets of probes as defined above, the sets being labelled with radioelements having a different emission energy, more preferably a distinguishable emission energy spectra. More preferably, the mean emission energy of the radioelements used should differ of at least 10 Kev, more preferably at least 20 Kev, even more preferably at least 30 Kev. Table 1 below discloses the emission energy, resolution and period for the preferred radioelements to be used in this invention.

**TABLE 1**

| Radioisotopes | emission | mean energy (KeV) | max. energy (KeV) | resolution (µm) | period |
|---|---|---|---|---|---|
| ³H | β⁻ | 5.7 | 18.6 | 0.5-5 | 12.3 years |
| ¹⁴C | β⁻ | 49.4 | 156.5 | 10-20 | 5730 years |
| ³⁵S | β⁻ | 48.8 | 167.5 | 10-15 | 87.4 days |
| ³³P | β⁻ | 76.4 | 248.5 | 15-20 | 25.6 days |
| ³²P | β⁻ | 695.5 | 1710.4 | 20-30 | 14.3 days |
| ¹²⁵I | e⁻ auger | 3.7 (79.3) | | 1-10 | 59.9 days |
| | | 22.7 (19.9%) | | | |
| | | 30.6 (10.7%) | | | |
| | | 34.5 (3.3%) | | | |
| | γ | 35.5 (6.7%) | | | |
| | X | 27.2 (39.6%) | | | |
| | | 27.4 (73.8%) | | | |
| | | 30.9 (21.3%) | | | |
| | | 31.7 (4.3%) | | | |

Table 1 shows that ³H emission energy spectrum is clearly distinguishable from that of ³⁵S, ³³P and ³²P, for instance. In a preferred embodiment, one set of probes is thus labelled with tritium and another set of probes is labelled with a radioisotope selected from ³⁵S, ³³P and ³²P. The examples disclosed below provide evidence that such sets of differently labelled probes can be used efficiently to simultaneously detect and discriminate target nucleic acids in a same biological sample, with a very high sensitivity.

Radioactive nucleotides to be used in this invention include natural and non-natural radiolabelled nucleotides, more preferably radiolabelled nucleotides selected from ATP, dATP, CTP, dCTP, GTP, dGTP, UTP, dUTP, TTP, dTTP. Such nucleotides are commercially available, or may be produced by conventional chemical methods. More preferred radiolabelled nucleotides to be used in the instant invention are listed in Table 2 below:

**TABLE 2**

| Isotope | Nucleotide | ref. | specif. Activity Ci/mmole |
|---|---|---|---|
| ³H | dATP | TRK 633 | 50-100 |
| | | TRK 347 | 1-10 |
| | dCTP | TRK 625 | 50-85 |
| | | TRK 352 | 15-30 |
| | dGTP | TRK 627 | 25-50 |
| | | TRK 350 | 5-20 |
| | dUTP | TRK 351 | 5-30 |
| ³⁵S | d ATPαS | SJ 1304,304,264, 1334, 1300 | 400-1000 |
| | dCTPαS | SJ 1305, 305, 1302 | 400-1000 |
| | UTPαS | SJ 1303, 603, 263 | 400-1000 |
| | (γ) ATP | BF1000 | ≥ 2500 |
| | (α) dATP | BF1001 | ≥ 2500 |
| ³³P | (α) dCTP | BF1003 | ≥ 2500 |
| | (α) CTP | BF1012 | ≥ 2500 |
| | (α) UTP | BF1002 | ≥ 2500 |
| ³²P | (α) dATP | PB10474, 10204, 10384, 10164 | 400-6000 |
| | (α) ATP | PB10200, 10160 | 400-3000 |
| | (γ) ATP | PB218, 168, 10218, 10168 | 3000-5000 |
| | (α) ddATP | PB10235, 10233 | 3000 - > 5000 |
| | (α) dCTP | PB10475, 10205, 10385,10165 | 400-6000 |
| | (α) CTP | PB10202, 20382, 10162, 40382 | 400-3000 |
| | (α) dGTP | PB10206, 10386, 10166 | 400-3000 |
| | (α) GTP | PB10201, 10161 | 400-3000 |
| | (γ)GTP | PB10244 | > 5000 |
| | (α) dTTP | PB10207, 10387, 10167 | 400-3000 |
| | (α) UTP | PB10163, 10203, 20383 | 400-3000 |
| ¹²⁵I | dCTP | NEX 074 | 2200 |

Even more preferably, radiolabelled nucleotides with high specific activity are being used, in order to produce probes with high specific activity value, as will be further disclosed below.

The probes may be radio-labelled according to different techniques.

### . Post-synthesis labelling

In a first embodiment, the probes are labelled post-synthesis. In this embodiment, the probes are first produced and then labelled, using a selected radio-isotope.

Post-synthesis labelling may be performed according to various strategies. In the preferred variant of this invention, the probes are labelled by addition of a terminal radioactive tracer to the probes. In a more preferred embodiment, the terminal radioactive tracer comprises one or several radioactive nucleotides having the same radio-isotope, i.e., a radioactive tail. The tail may be a homopolymer, i.e., composed of the same repeated nucleotide, or a heteropolymer, i.e., composed of several different nucleotides. Where a heteropolymer tail is used, the sequence should preferably be determined so as not to interfere with the hybridisation of the probe and not to form secondary structures (loops, etc.).

In a preferred embodiment, the terminal radioactive tracer is a homopolymer tail, more preferably a 3'(homopolymer)-tail.

Furthermore, in the tail, all or only a part of the nucleotides may be radio-labelled. Indeed, by adapting the concentration or proportion of radioactive nucleotides in the tail, it is possible to control or adjust the specific activity of the probe. Obviously, the radioactive nucleotides present in the tail should preferably all bear the same radio-isotope so that each set of probes is characterized by a particular radioisotope.

The specific activity of the probes may be further adapted by controlling or adjusting the length of the tail. In this regard, in a particular embodiment of this invention, the tail comprises preferably 5 to 100 nucleotides, more preferably between 5 and 50 nucleotides, even more preferably between 5 and 30 nucleotides, and even more preferably at least 25% of the nucleotides in the tail are radiolabelled.

The tail may be produced either separately and then linked to the probe, or by direct sequential addition of the nucleotides to the probe.

In this regard, in a preferred embodiment, the probe is labelled by contacting the probe with radioactive nucleotides in the presence of an enzyme that catalyses the 3' binding of nucleotides. A typical enzyme to be used is a terminal transferase. As indicated above, the concentration of the nucleotides and the proportion of radioactive and non radioactive nucleotides may be adapted to adjust the specific activity of the probe.

In a preferred variant, the probe comprises a 3'-tail produced by sequential addition to the probe of 5-100 nucleotides, all or part of which bearing a selected radiolabel. More preferably, the 3' tail is a 5-100 bases long homopolymer, preferably a polyA, polyC, polyG, polyT or polyU tail, in which all or part of the nucleotides bear a selected radioisotope.

Post synthesis labelling may also be performed by addition of radiolabelled phosphates (e.g., (γATP, γGTP)³²P, γATP³³P, ³⁵S-thio-phosphates) to the 5' end of the probes, using suitable enzymes such as T4 kinase. Such method may be used alone or in combination with others, since it may not allow very high specific activity to be achieved.

### . Labelling during synthesis

The probes can also be labelled during their synthesis. In this embodiment, radiolabelled nucleotides are incorporated into the probe during the synthesis. This embodiment is particularly suited for RNA probes which are produced in in vitro transcription systems as mentioned above. As for post-synthesis labelling, the specific activity of the probe can be adjusted by controlling the concentration of radiolabelled nucleotide in the synthesis medium.

In a preferred embodiment, each set of probes to be used in the same assay should be labelled using the same technique (i.e., post-synthesis or during synthesis, 3' tail vs 5' phosphate, etc). Even more preferably, to perform the present invention, the probes of each set contain a 3'-tail, more preferably a 3'-homopolymer tail, even more preferably a 3'-homopolymer tail comprising between about 15 and about 85 nucleotides.

### . Non-radioactive probes or labelling

While the invention discloses methods of detecting (or quantifying or visualizing) nucleic acids in samples using at least two differently radiolabelled sets of probes, it should be understood that the invention may be performed by combining said radiolabelled probes with any other probe or detection reagent, in order to obtain a further detailed image of the sample.

In this regard, additional non-radioactive probes may be used, such as fluorescent probes, in combination with the above radioactive probes, so that additional genes or RNAs can be monitored simultaneously in the sample, or to introduce additional controls.

Also, additional detection reagents, such as affinity reagents, may be used in order to further detect proteins (or polypeptides), receptors, organelles, etc. within the sample. Such reagents include immunomolecules such as antibodies (or fragments or derivatives thereof), which can be labelled according to conventional techniques (enzymatic, fluorescent, chemical, etc.).

### The biological sample

The biological sample may be any mammalian biological material such as tissue sample, organ sample, biopsy, skin sample, biological fluid, bone marrow, nervous tissue (e.g., brain tissue), etc. The biological material may also comprise plant tissue or cells, prokaryotic cells, lower eukaryotic cells, established cell cultures, viruses, any other unicellular organism, etc. Because of the high sensibility and high reproducibility of the present method, very low quantities of biological material may be used, and the invention can be applied to essentially all types of biological material. The invention is particularly suited for detecting rare mRNA species as well as fine gene expression regulation within complex tissues, such as nervous tissue.

The biological sample of a mammalian or plant tissue is typically prepared by cutting fresh-frozen tissues on a cryostat, for example 10-15 µm thick sections.

Alternatively, the biological sample may be prepared from any tissue by fixation in suitable substances such as paraffin. The tissue may then be cut in a vibratome to produce appropriate section.

The biological material is preferably deposited on a support prior to the contacting with the probes. The support may be any suitable support for genetic analysis, including plastic, nylon, glass, silicium, etc. A typical example of glass slide includes the SuperFrost^{R} Plus (Menzel-Glaser, Germany). Preferably, the support comprises glass, such as glass slides. The support may be pre-treated to ensure adhesion or immobilization of the biological sample thereto (e.g., gelatine-coated). The biological sample (or the support) may then be stored for later analysis, or use directly. Where storage is performed, freezing may be used, such as freezing at-20°C or -80°C, for instance, preferably after air drying.

In a preferred variant of this invention, several biological samples are tested in parallel. The various samples may be deposited on the same support, or on separate supports. In a preferred embodiment, several samples are deposited on the same support. The samples may be different sections of a same tissue, a tissue sample or cell population at various stages (maturation, treatment with a compound, apoptotic, cancerous, etc.) ; different samples of the same tissue or cell population from different origins (e.g., different subjects, different species, etc.). As indicated before, it is believed that the instant invention can be used with essentially any biological sample and should not be limited to particular applications. Preferably, the sample is a mammalian tissue sample, in particular a human tissue sample, such as nervous cells, blood cells, tumor cells, embryonic cells, etc.

Prior to contacting the biological material(s) with the probes, the biological material(s) may be subjected to various pretreatments, such as fixation, permeabilization, delipidation, etc. In a preferred embodiment, the sample is subjected to fixation, using conventional agents. Fixation allows to maintain the sample in its status (e.g., to avoid RNA degradation, protease activity, nuclease activity, etc.). Preferably, the sample is fixed using formaldehyde, paraformaldehyde (PFA), glutaraldehyde, Bouin solution, etc.. More preferably, the samples are subjected to fixation in the presence of a PFA solution (e.g., 4%). For samples that are not frozen (e.g., in paraffin), they may be subjected to fixation prior to their deposit on the support.

While additional pretreatments may be performed, the invention can be used efficiently with no need for further treatments such as permeabilization, especially with frozen samples. This represents another advantage of the instant invention. Where samples in paraffin are used, they are preferably treated with protease to increase permeability.

### Hybridisation

The present invention now provides, for the first time, evidence that differently labelled sets of probes can be used simultaneously on a biological sample and that the signals emitted can be discriminated. The invention demonstrates that the discrimination can be made by adapting the specific activity of the probes and controlling the hybridisation conditions, as will be discussed below.

In the present invention, the sample is contacted with at least two sets of probes as defined above. The contacting allows formation of hybrids between the nucleic acids of the sample and the probes, where target nucleic acid is present in the sample. Accordingly, the contacting shall be made under conditions sufficient to allow nucleic acid hybridisation to occur. Conditions for forming hybridisation have been disclosed for instant in Maniatis et al (Molecular Cloning, a Laboratory Manual, 1989) or in Nucleic Acid Hybridization, A practical approach IRL Press, Wash. DC (1985).

In this regard, in order to ensure high sensitivity of the method, the contacting step is preferably performed under conditions allowing the probes to hybridise with the target nucleic acid as well as, potentially, with non-target (i.e., aspecific) nucleic acids, non-specific hybridisation being eliminated or reduced by suitable washing conditions. The hybridisation condition can be adjusted by the skilled artisan. Essentially, hybridisation can be controlled by the hybridisation medium and temperature. In this respect, hybridisation is preferably performed at temperatures between about 30 and about 70 °C (high temperatures 60-70°C being preferred for RNA probes). Furthermore, the hybridisation medium generally comprises standard saline citrate solution (SSC) at moderate saline strength. Specific hybridisation conditions are disclosed in the examples and can be adapted by the skilled person. Typically, the hybridisation medium comprises Denhardt's solution and SSC solution. Furthermore, the hybridisation medium may comprise additional agents that reduce non-specific signal or probes rearrangements, for instance. In this respect, the hybridisation medium generally comprises dithiothreitol (DTT) and/or formamide. In addition, in a particular aspect of this invention, hybridisation is performed in the presence of competitor nucleic acid, to reduce background signal. In particular, where the probes contain a labelled nucleotide tail, the contacting step can be performed in the presence of un-labelled oligonucleotides complementary to the tail. The competitor nucleic acid may be used simulatenously with the probes, or contacted with the sample prior to the probe.

A preferred hybridisation medium thus comprises SSC, DDT, formamide and a competitor nucleic acid.

In a typical experiment, each biological sample is contacted with a hybridisation medium in the presence of at least two radioactive sets of probes, for a period of time sufficient to ensure formation of hybrids, for instance between 1 hour to 12 hours.

In order to allow efficient discrimination and visualization of each set of probes (i.e., each target nucleic acid) on the sample(s), it is preferred to use particular amounts of sets of probes, with a particular specific activity, for the hybridisation step. In this regard, the invention now demonstrates that efficient discrimination (and quantification) of the different labels is best achieved where both sets of probes have a specific activity comprised between about 5.10⁷ and 5.10¹⁰ cpm/µg, more preferably between about 10⁸ and 10¹⁰ cpm/µg, even more preferably between about 5.10⁸ and 5.10⁹ cpm/µg. A more preferred way of performing the invention comprises the use of two sets of probes having essentially the same specific activity, i.e., not differing by more than about 3 times from each other(s), more preferably not by more than about two times. The specific activity of the probes can be adjusted by the choice of the nucleotide (see table 2 above) and the conditions of the labelling method, as discussed above. In this respect, where the selected radionucleotides have a distinct specific activity, the labelling conditions should be adjusted to ensure that the labelled probes have essentially a similar specific activity.

In addition, in performing the hybridisation, it is also recommended to use similar amounts of each set of probes, so that more reliable and comparable results are obtained. In this regard, typical experiments are performed using between 0.05 and 0.5 pmoles of probes of each set, more preferably between 0.05 and 0.2 pmole. While these are preferred conditions allowing discrimination of nucleic acids present at very broad spectrum of levels (i.e., from rare to very abundant) and from virtually any type of biological material, it should be understood that the molarity (or amount) of probes of each set can be adjusted by the skilled artisan to the specific conditions or biological samples.

The present invention can be implemented using a variety of nucleic acid probes, as described above. These probes may vary in length as well as in nature. In this regard, it is possible to use, in performing the invention, two nucleic acid probes of the same or different nature. More particularly, the nucleic acid probes may be either both oligonucleotides, DNAs, RNAs, PNAs, etc. (i.e., of the same nature) or of a different nature, e.g., oligonucleotide probes and DNA probes, oligonucleotide probes and RNA probes, DNA probes and RNA probes, etc. Generally, any probe mixture or combination can be used in the present invention.

In order to perform simultaneous (in situ) hybridisation of differently radiolabelled probes, each labelled set of probes is contacted simultaneously with the sample.

The term "simultaneously" indicates that the hybridisation should be performed with the two sets of probes essentially at the same time, so that only one hybridisation/washing round in performed. However, "simultaneous" does not require that the sets of probes be contacted with the sample at exactly the same time.

In a particular embodiment, the sets of probes are mixed with the hybridisation medium, and the samples are then exposed to the resulting solution.

In another embodiment, the samples are first exposed to the hybridisation medium, and the sets of probes are then added, either simultaneously or sequentially.

Typically, between 20 to 200 µl of hybridisation medium is added to each sample. The exposure time may vary, for instance, from 1 or several hours to one or several days. Preferably, the hybridisation lasts for less than about 24 hours, typically between 1 and 12 hours.

The samples are then rinsed to eliminate unbound probes as well as non-specific hybridisation. In this regard, any conventional washing solution may be used, such as saline solutions. Preferably, the samples are washed using saline citrate solution (SSC) comprising DTT, in order to eliminate non-specific hybrids formed. Preferred washing conditions use DTT (e.g., 10 mM) at elevated temperatures, typically 10-20°C below the theoretical melting temperature, preferably above 40°C, more preferably above 45°C, in a specific example above about 50°C. Several washings may be performed to increase the selectivity of the method.

The samples are then preferably dried (e.g., dehydrated) and apposed to scintillating paper for subsequent measure of the radioactivity (readout).

### Readout

In order to assess hybrid formation on the samples and to detect the presence (or amount) of target nucleic acids in said samples, the method comprises (i) washing the unbound probe (as described above) and (ii) detecting radioactivity (i.e., the first and second radiolabel) on the sample.

Radioactivity detection and discrimination may be achieved by different techniques using quantitative imaging devices such as Beta Imager (50-250 µm depending on the radioisotope used) and the Micro Imager that provides direct detection by the solid scintillator sheet principle and allows cellular size expression analysis (15 µm).

Preferably, acquisition of radioactive images is performed with a Micro Imager (Biospace Mesures, Paris, France), a real time, high-resolution digital autoradiography system. The instrument allows precise quantitative imaging of tissue section with a spatial resolution of 15 µm and a pixel size of 5 µm. Imaging is performed by optical contact between the radiolabeled sample, a thin foil of scintillating paper, and an intensified CCD camera. Beta particles are identified through light spot emission in the scintillating foil, allowing thus filtering of the background noise as well as filtering of emissions due to isotopes of different energies (FR2,772,484). The instrument is particularly well suited to the imaging and quantification of dual labelled samples and in particular to the simultaneous measurement of differential gene expression.

Imaging is performed on a 24 mm x 32-mm area. An automated sample feeder allows successive imaging of up to four slides. Detection threshold is kept to the very low level of 0,4 counts per minute per square millimetre for tritium labelling, and ten times lower for higher energy isotopes, a figure obtained thanks to the intrinsic noise suppression of the instrument. Because of the direct particle counting principle of the instrument, quantification is obtained with a precision better than 5%, without underexposure or saturation effects over four decades. Very fine variations of gene expression levels can therefore be measured with high accuracy.

In a preferred embodiment, radioactivity detection is thus performed by optical contact between the labelled sample, a thin foil of scintillating paper and an intensified CCD camera.

The invention can be used to detect gene expression in any biological sample, for research, diagnostic or any other experimental or industrial applications (pharmacogenomics, etc). Gene expression may be used to identify a dysfunction, compare gene regulation, identify therapeutic genes, assess responsiveness of a subject, assess the presence of pathogenic agents (e.g., virus, bacteria, etc.) in a sample, etc.

Other aspects and advantages of the present invention will be described in the following examples, which should be regarded as illustrative and not limiting the scope of protection.

### LEGEND TO THE FIGURES

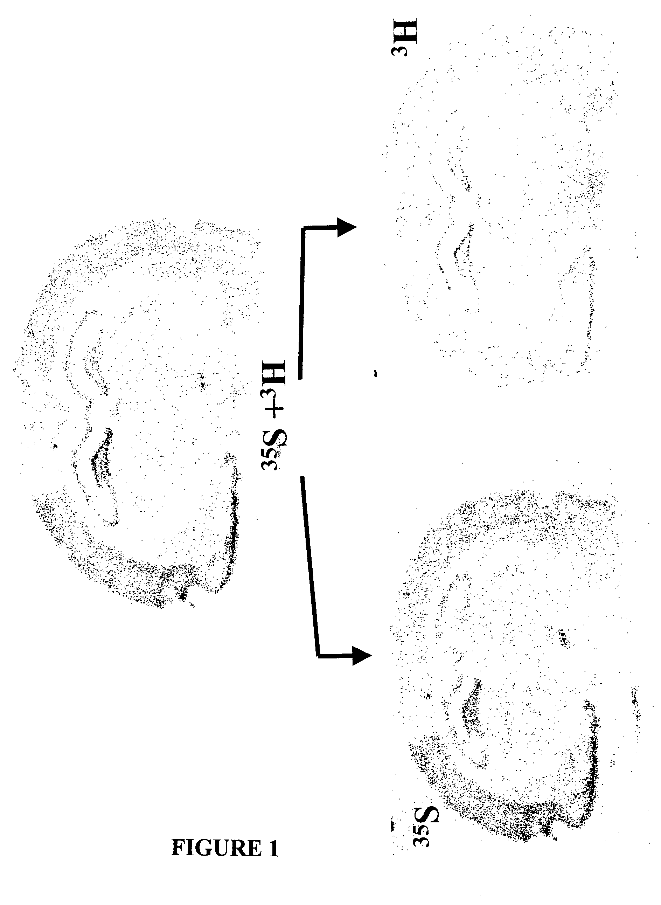

Figure 1: Detection and discrimination of radiolabelled probes on coronal sections.

### EXAMPLES

### Example 1

### Tissue preparation

Coronal sections were cut at 15-20 µm in the dorsal part of the dentate gyrus at-20°C on a Leitz cryostat. Sections were mounted onto superfrost treated slides, air dried and stored at -80°C until required. Frozen sections were first warmed to room temperature and then fixed in 4% paraformaldehyde in phosphate buffered saline (PBS, pH 7.4) for 20 min at room temperature, washed in PBS for 3 times (5 min), dried in absolute ethanol.

### DNA probe preparation

Antisense oligonucleotides were synthesized in-house on a Beckman Oligo 1000DNA synthesizer. Oligonucleotide sequences were designed complementary to rat mRNA-derived sequences available in published databases. The used probes were oligonucleotides complementary to specific regions of syntaxin 1B sequence (35-mer oligonucleotide sequence: 5'-GAT GTG TGG GGA GGG TCC TGG GGA AGA GAA GGG TA-3') and Homer sequence (39-mer oligonucleotide sequence: 5'-GGT CAG TTC CAT CTT CTC CTG CGA CTT CTC CTT TGC CAG-3').

Probes were 3'end-labelled with α-³⁵S-deoxyadenosine triphosphate (³⁵S-dATP, SJ 1334, Amersham) or deoxy[1',2',5,³H]cytidine 5' triphosphate (³H-dCTP,TRK.625, Amersham) in a tailing reaction, using terminal deoxynucleotide transferase (Amersham). 60 ng of each oligonucleotide was incubated in 40 µl buffer solution containing 8 µl of terminal transferase buffer x5 (M189A, Promega), 4 µl of ³⁵S-αdATP or 40 µl of ³H-dCTP (previously dehydrated and dissolved in 4 µl of distillated water) and 2 µl of terminal deoxynucleotide transferase ( E2230Z, Amersham). Purification of the labelled probes was performed on P10 column (150-4140, Biorad). The specific activity of each labelled probe was between 4x10⁸ and 9x10⁸ cpm/µg.

### In situ hybridisation

Sections were post-fixed in 4% paraformaldehyde in phosphate-buffered saline (PBS) immediately before hybridisation. The hybridisation solution was composed for 1 ml of:

| | |
|---|---|
| 500 µl deionized formamide | (50%)_{f} |
| 20 µl Denhart 50 | (X1)_{f} |
| 200 µl SSC X20 | (X4)_{f} |
| 100 µl DTT 1M | (100 mM)_{f} |
| 100 mg Dextran sulfate | (10%)_{f} |
| 25 µl yeast tRNA 10mg/ml | (250 mg/ml)_{f} |
| 25 µl poly A 10 mg/ml | (250 mg/ml)_{f} |
| 25 µl herring sperm DNA 10 mg/ml | (250 mg/ml)_{f} |

The two probes were simultaneously diluted to 1/100 with the hybridisation solution and 70 µl of the mixture were applied to each brain slice. Sections were incubated overnight at 50°C under parafilm Fuji, then washed twice for 15 min in 1X standard saline citrate (SSC)/10mM DTT at 53°C, twice for 15 min in 0.5X SSC/10mM DTT at 53°C and once in 0.5X SCC/10mM DTT at room temperature before being dried by dipping into an ethanol bath. Control experiments were performed either by displacing specific mRNA hybridisation by a 50-fold excess of unlabelled oligonucleotides or by using a sense oligonucleotide that yielded no signal in tissue sections.

### Double labelling in in situ hybridisation.

Acquisition of radioactive images was performed with a Micro Imager (Biospace Mesures, Paris, France) for 15 hours. The whole dentate gyrus of the brain slice was delimited and the number of desintegrations per area of this region was measured using β-Vision software (Biospace). The results are presented on Figure 1 and clearly demonstrate simultaneous visualization of both target nucleic acids in the tissue sample.

### Example 2

A dual detection method was performed essentially as described in Example 1, using the following hybridisation solution: hybridisation solution (Amersham, UK) supplemented with 40% v/v deionised formamide (MERCK), 50 µg/ml poly A⁺ (Sigma) and 50 mM 4-dithriothreitol (DDT, Euromedex).

The results obtained allowed the detection and discrimination of target nucleic acids of the samples.

### Example 3

A dual detection method was performed essentially as described in Example 1, except that one set of probes was 3'-end labelled with deoxy[1',2',5,³H]cytidine 5' triphosphate (³H-dCTP,TRK.625, Amersham) and the other was 3'-end labelled with ³³P(α)dATP (BF1001), in a tailing reaction, using terminal deoxynucleotide transferase (Amersham).

The results obtained allowed the detection and discrimination of target nucleic acids of the samples.

## Claims

1. A method of detecting target nucleic acids in a biological sample, comprising:
a) contacting the biological sample with at least two sets of radioactive probes, the probes of the first set being specific for a first target nucleic acid and labelled with a first radio-label, and the probes of the second set being specific for a second target nucleic acid and labelled with a second radio-label, and
b) detecting said first and second target nucleic acids in the biological sample by assessing the formation of hybrids between the probes and the sample.

2. The method of claim 1, wherein the probes are DNA molecules between 15 and 2000 base-pair long, more preferably between 15 and 500 base-pairs-long.

3. The method of claim 2, wherein the probes are single stranded DNA oligonucleotides between 15 and 500 bases long.

4. The method of claim 1, wherein the probes are RNA molecules, between 15 and 3000 bases long.

5. The method of any one of the preceding claims, wherein the probes are labelled by a 3' radioactive tracer, preferably a 3', 5-100 long, radiolabelled nucleic acid tail.

6. The method of any one of claims 1 to 5, wherein the probes are labelled by a 5' radioactive tracer.

7. The method of any one of claims 1 to 6, wherein the probes comprise, in their sequence, radioactive nucleotides.

8. The method of any one of the preceding claims, wherein the two sets of probes are comprised of nucleic acids of the same nature, for instance oligonucleotide probes.

9. The method of any one of claims 1 to 7, wherein the two sets of probes are comprised of nucleic acids of a different nature.

10. The method of any one of the preceding claims, wherein the probes of the two sets comprise a 3' radioactive tracer.

11. The method of any one of the preceding claims, wherein the first and second radiolabel have a different emission-energy spectra.

12. The method of claim 11, wherein the first set of probes is labelled with tritium and the second set of probes is labelled with a radioisotope selected from ³⁵S, ³³P, ³²P and ¹²⁵I.

13. The method of any one of the preceding claims, wherein the two sets of probes are contacted simultaneously with the biological sample.

14. The method of any one of the preceding claims, wherein the two sets of probes have essentially the same specific activity.

15. The method of any one of the preceding claims, wherein essentially the same amount of the two sets of probes is used.

16. The method of any one of the preceding claims, wherein the biological sample is a mammalian tissue sample, preferably a tissue section.

17. The method of any one of the preceding claims, wherein several biological samples are contacted in parallel.

18. The method of any one of the preceding claims, wherein the samples are deposited on one or several supports, preferably glass support.

19. A method of detecting target nucleic acids in several biological samples, comprising:
a) providing biological samples on one or several supports, preferably glass supports,
b) contacting, in parallel, the biological samples on the support(s) with at least two sets of radioactive probes, the probes of the first set being specific for a first target nucleic acid and labelled with a first radio-element, and the probes of the second set being specific for a second target nucleic acid and labelled with a second radio-element, and
c) detecting said first and second target nucleic acids in the biological samples by assessing the formation of hybrids between the probes and the samples.

20. A method of detecting target nucleic acids in a biological sample, wherein the biological sample is contacted, in parallel with the following at least two sets of probes:
a) probes of a first set specific for a first target nucleic acid and labelled with a first radio-label and probes of a second set specific for a second target nucleic acid and labelled with a second radio-label,
b) probes of the first set specific for the first target nucleic acid and labelled with the second radio-label and probes of the second set specific for the second target nucleic acid and labelled with the first radio-label,
and wherein the method further comprises assessing the formation of hybrids between the probes and the samples.

21. A method for comparing target gene expression in at least two biological samples, comprising:
a) contacting, in parallel, the biological samples with at least two sets of radioactive probes, the probes of the first set being specific for a first target nucleic acid and labelled with a first radio-element, and the probes of the second set being specific for a second target nucleic acid and labelled with a second radio-element,
b) assessing the formation of hybrids between the probes and the samples, and
c) comparing target gene expression in said samples by comparing the relative amount of hybrids formed between the samples.

22. The method of any one of the preceding claims, wherein one of the sets of probes is specific for a control reference nucleic acid.

23. The method of any one of the preceding claims, wherein assessing hybrid formation comprises (i) washing the unbound probe and (ii) detecting radioactivity on the sample.

24. A RNA molecule or set of probes, wherein the RNA molecule or set of probes comprises radioactive nucleotides labelled with tritium.

25. An isolated nucleic acid molecule, wherein the molecule is single strand, comprises a 15-100 bases-long sequence which is complementary to a target nucleic acid, and comprises a 3' tritiated nucleotide tail.

26. The use of two radioactive probes with different specificity and different radioactive label, for in vitro or ex vivo gene expression analysis on a biological sample.

27. A method of any one of claims 1 to 23, further comprising contacting the biological sample(s) with a non-radioactive probe and/or an affinity reagent to detect additional target nucleic acid(s), polypeptide(s) or cellular component(s).

28. A method for simultaneous detection (or quantification) of at least two target components of a cell or tissue (including nucleic acid, polypeptide, organelle) using two differently radiolabelled detection reagents.

29. A kit for gene detection comprising radioactive nucleotides, enzymes and/or protocols for radioactive labelling of nucleic acid probes.

30. A kit for implementing a method according to any one of claims 1 to 23, 27 and 28, comprising the reagents, supports and/or protocols for labelling, hybridisation and/or readout.
